# EUROPÄISCHE PATENTSCHRIFT

(11) **EP 1 531 886 B1**
(45) Veröffentlichungstag und Bekanntmachung des Hinweises auf die Patenterteilung: **08.06.2011**
(21) Anmeldenummer: 03794947.6
(22) Anmeldetag: 27.08.2003
(51) Int. Cl.: A61M 5/00

(54) **VERABREICHUNGSVORRICHTUNG MIT TEMPERATURSENSOR**
ADMINISTERING DEVICE COMPRISING A TEMPERATURE SENSOR
DISPOSITIF D'ADMINISTRATION POURVU D'UN CAPTEUR DE TEMPERATURE

(30) Priorität: 31.08.2002 CH 148802; 03.09.2002 US 407812 P
(43) Veröffentlichungstag der Anmeldung: 25.05.2005
(73) Patentinhaber: F. Hoffmann-La Roche AG, 4070 Basel (CH); Roche Diagnostics GmbH, 68305 Mannheim (DE)
(72) Erfinder: KIPFER, Urs, 3432 Luetzelsflueh (CH)
(74) Vertreter: Wess, Wolfgang
(86) Internationale Anmeldenummer: PCT/EP2003/009476
(87) Internationale Veröffentlichungsnummer: WO 2004/024217

(56) Entgegenhaltungen:
- EP-A- 0 824 022
- WO-A-96/32975
- US-A- 6 129 702
- US-A1- 2001 034 502
- US-B1- 6 375 624

## Beschreibung

Die Erfindung betrifft eine Vorrichtung zur Verabreichung eines fluiden Produkts. Insbesondere betrifft die Erfindung ein Infusionsgerät zur Verabreichung von Insulin.

Derartige Vorrichtungen werden z. B. in medizinische oder therapeutischen Anwendungen eingesetzt, um wiederholt oder kontinuierlich eine Produktdosis mit einer bestimmten Dosismenge eines fluiden Produkts zu verabreichen. Das fluide Produkt kann z. B. eine flüssige Wirkstofflösung, wie etwa Insulin oder Wachstumshormone, sein. Die Verabreichungsvorrichtungen können als stationäre Geräte oder als tragbare Geräte, die von einem Anwender z. B. am Körper mitgeführt werden, ausgebildet sein.

Aus der US 6,129,703 ist ein Medikamentendosierungssystem mit einem Temperatursensor zum Detektieren der Temperatur eines flüssigen Medikaments bekannt. Der Sensor gibt die Temperatur an eine Kontrolleinheit weiter, die aufgrund dieser Informationen eine Ventileinstellvorrichtung anregt, die Durchflussrate des Medikaments in Abhängigkeit von der detektierten Medikamententemperatur zu verstellen. Die Ventilstellvorrichtung der US 6,129,703 reagiert unmittelbar auf die Signale der Kontrolleinheit, die wiederum kontinuierlich die aktuellen Temperaturinformationen von dem Sensor erhält. Die US 6,375,624 B1 behandelt die Entdeckung von Flüssigkeitsaustritten bei Fluidinjektionen. Dazu werden beispielsweise kontinuierlich die Körpertemperatur des Patienten an der Verabreichungsstelle und die Temperatur des Medikaments gemessen. Aus dem Gewebetemperatursignal und dem Flüssigkeitstemperatursignal wird eine Funktion gebildet, die Rückschlüsse auf die Wahrscheinlichkeit des Auftretens von Flüssigkeitsaustritten zulässt. Dazu wird ein kalkulierter Grenzwert, bei dem ein Flüssigkeitsaustritt wahrscheinlich ist, mit dem ermittelten Messwert verglichen. Aus der EP 0 824 022 A1 ist ein parenterales Medikamentenabgabesystem bekannt. Es dient der zeitweisen Erhöhung der Medikamentendosis bei sehr kleinen Verabreichungsmengen. Ein Temperatursensor kommt zum Einsatz, um Änderungen der Temperatur zu detektieren, die zum Beispiel Einfluss auf die Viskosität der Flüssigkeit haben können. Die WO 96/32975 beschreibt ein Gerät zum Injizieren eines Kontrastmittels. Das Gerät verfügt über einen Temperatursensor, der bei der Feststellung, dass das zu verabreichende Mittel zu warm ist, die Zufuhr zum Patienten sofort unterbricht. Ist das Mittel dagegen zu kalt, so wird eine Heizung aktiviert, um das Mittel auf die ideale Temperatur zu bringen. Zur Bestimmung, ob die Temperatur zu hoch oder zu niedrig ist, wird die aktuelle Temperatur mit einer Referenztemperatur verglichen.

Aus der EP-B-0 143 895 ist z. B. eine Infusionspumpe bekannt, die zur Infusion einer Wirkstofflösung dient. Die Infusionspumpe umfasst ein Gehäuse, in dem ein Behälter für die Wirkstofflösung, eine Verabreichungseinrichtung zur Verabreichung der Wirkstofflösung aus dem Behälter und eine Dosiereinrichtung zur Einstellung einer zu verabreichenden bestimmten Produktdosis aufgenommen ist. In dem Behälter ist ein Stopfen vorgesehen, der zur Ausschüttung der Wirkstofflösung innerhalb des Behälters auf einen Auslass hin in Vorschubrichtung verschiebbar aufgenommen ist. Die Verabreichungseinrichtung umfasst ein Abtriebsglied und eine Antriebseinrichtung. Das Abtriebsglied schließt sich an den Stopfen in dem Behälter an und wird zum Vorschub des Stopfens innerhalb des Behälters von der Antriebseinrichtung angetrieben. Durch die Dosiereinrichtung wird eine Dosis mit einer gewünschten Dosismenge des Wirkstoffs eingestellt. Die Einstellung erfolgt z. B. durch eine der Dosismenge entsprechenden Begrenzung des Vorschubwegs des Stopfens innerhalb des Behälters. Die Antriebseinrichtung kann von Hand betätigt werden oder es kann ein Elektromotor vorgesehen sein, der als Antrieb für das Abtriebsglied dient. Der Vorschub des Abtriebsglieds, bzw. des Stopfens innerhalb des Behälters, kann auch durch einen Drehmechanismus erfolgen, wobei eine Drehung in eine translatorische Bewegung zum Antrieb des Stopfens umgewandelt wird. Die Verabreichung der Wirkstofflösung, bzw. des fluiden Produkts, erfolgt derart, dass bei Betätigung der Verabreichungseinrichtung der Stopfen um eine eingestellte Weglänge in Richtung eines Behälterauslasses verschoben wird und dadurch Wirkstoff z. B. in einen Infusionsschlauch abgegeben und in einen Patienten über einen Zugang z. B. in Form einer Kanüle, eingeführt werden kann.

Solche Verabreichungsvorrichtungen sind im Allgemeinen teilweise vorprogrammiert, d. h. die Verabreichung des fluiden Produkts wird über eine Speicher- und Steuereinheit kontrolliert, durch die z. B. die Größe einer Dosismenge und die Verabreichungsfrequenz bestimmt werden. Ferner können herkömmliche Verabreichungsvorrichtungen verschiedene Kontroll- und Anzeigeeinrichtungen besitzen, um eine Überwachung der Produktverabreichung zu ermöglichen. Hierfür werden z. B. Messsysteme zur Aufnahme verschiedener Körperparameter eines Patienten eingesetzt, wie etwa die Messung der Pulsrate, des Blutdrucks, des Blutzuckergehalts, oder der Körpertemperatur. Diese Parameter können dann zur Steuerung der Verabreichung an die Speicher- und Steuereinheit weitergegeben werden. Dadurch kann z. B. die Ausschüttgenauigkeit des fluiden Produkts verbessert werden oder es können Okklusionen oder Leckagen innerhalb eines Leitungssystems für das fluide Produkt detektiert werden.

Bisher blieben bei der Steuerung solcher Verabreichungsvorrichtungen jedoch sich verändernde Parameter des fluiden Produkts außer Betracht. Zum Beispiel wird die Ausschüttgenauigkeit durch Temperaturschwankungen beeinflusst, denen die Verabreichungsvorrichtung und damit das fluide Produkt unterliegt. Das fluide Produkt kann je nach Temperatur unterschiedliche Zustandsformen aufweisen. Beispielsweise kann ein Lösungsmittel, in dem ein Wirkstoff gelöst ist, bei höheren Temperaturen ein höheres Volumen aufweisen, so dass sich die Konzentration des Wirkstoffs in der Lösung ändert. Ferner kann der Wirkstoff selbst seine Eigenschaften entsprechend einer bestimmten Temperatur ändern. Durch eine Veränderung des fluiden Produkts mit der Temperatur verändert sich auch die Menge des Wirkstoffes, der bei der Verabreichung einer eingestellten Produktdosis abgegeben wird. Dadurch kann einem Patienten zu viel oder auch zu wenig Wirkstoff zugeführt werden.

Messungen haben z. B. gezeigt, dass es für Insulin bei Temperaturschwankungen mit Temperaturdifferenzen von 15°C bei einem Behältnis von z. B. 300 Einheiten zu einer Ungenauigkeit von ± 1 Einheit führen kann. Eine derartige Temperaturschwankung kann z. B beim Zubettgehen oder beim kurzen Verweilen an der Sonne auftreten. Viele Diabetiker haben z. B. eine durchschnittliche Insulinförderrate von 0,5 Einheiten pro Stunde. Bei einer temperaturabhängigen Ungenauigkeit einer verabreichten Dosismenge kann es daher leicht zu einer Fehldosierung des Insulins und damit zu einer Fehlbehandlung des Patienten kommen.

Es ist daher eine Aufgabe der vorliegenden Erfindung, eine Vorrichtung zur Verabreichung eines fluiden Produkts zu schaffen, bei dem eine Dosiseinstellung und eine Abgabegenauigkeit einer Produktdosis verbessert wird, bei dem Temperaturschwankungen berücksichtigt werden und eine zuverlässige Funktion der Verabreichungsvorrichtung gewährleistet wird.

Die Aufgabe wird durch eine Vorrichtung zur Verabreichung eines fluiden Produkts nach Anspruch 1 gelost. Vorteilhafte Ausgestaltungen der Vorrichtung gehen aus den Unteransprüchen hervor.

Demnach umfasst eine Vorrichtung zur Verabreichung eines fluiden Produkts, etwa einer flüssigen Wirkstofflösung, einen Behälter für das fluide Produkt, eine Abgabeeinrichtung zur Verabreichung des fluiden Produkts aus dem Behälter und eine Dosiereinrichtung zur Einstellung einer Produktdosis. Der Produktbehälter kann z. B. durch eine herkömmliche Ampulle gegeben sein oder das fluide Produkt kann in eine Kammer der Verabreichungsvorrichtung, die als Behälter dient, eingefüllt werden. Der Behälter weist wie oben beschrieben, einen Auslass zur Abgabe des Produkts aus dem Behälter und einen Stopfen auf, der von der Abgabeeinrichtung in Vorschubrichtung bewegt werden kann. Die Dosiereinrichtung weist vorzugsweise einen Drehmechanismus auf, bei dem zur Einstellung einer Produktdosis durch die Einstellung einer bestimmten Drehposition eines Drehgliedes, der Vorschubweg des Stopfens innerhalb des Behälters begrenzt wird. An dem Behälterauslass schließt sich eine Ableitung zur Abgabe des fluiden Produkts an. Hierfür kann z. B. ein Schlauch und eine Kanüle vorgesehen sein, die auf einer Oberfläche eines Körpergewebes des Patienten angeordnet ist und in dieses hineinführt.

Erfindungsgemäß weist die Verabreichungsvorrichtung wenigstens einen Temperatursensor auf, der die Temperatur des fluiden Produkts in dem Behälter und/oder die Temperatur in der Nähe des fluiden Produkts, bzw. des Behälters, misst. Vorzugsweise ist der wenigstens eine Temperatursensor hierfür an oder in dem Behälter angeordnet. Es können auch mehrere Temperatursensoren vorgesehen sein, die an unterschiedlichen Orten an oder in dem Behälter, bzw. der Verabreichungsvorrichtung, vorgesehen sind, um z. B. durch einen Temperaturvergleich eine genaue Temperaturmessung zu ermöglichen.

Zur Steuerung einer Verabreichung des fluiden Produkts aus der oben beschriebenen Verabreichungsvorrichtung weist die Verabreichungsvorrichtung eine Steuereinheit mit einem Speicher und z.B. einer Batterie auf, durch die mit Hilfe einer Dosiereinrichtung eine Dosismenge eines Produkts, die bei einer Verabreichung abgegeben werden soll, eingestellt werden kann. Die Einstellung der Dosismenge erfolgt erfindungsgemäß in Abhängigkeit von der Temperatur des fluiden Produkts und/oder in Abhängigkeit von der Temperatur der Umgebung nahe des Behälters oder auch nahe der Verabreichungsvorrichtung. Für ein nicht beanspruchtes Verabreichungssteuerverfahren kann z. B. ein von einem Temperatursensor gemessener Temperaturwert an die Steuereinheit abgegeben werden, woraufhin die Steuereinheit die Einstellung der Dosismenge mittels der Dosiereinrichtung bestimmt und die Dosiereinrichtung entsprechend ansteuert, bzw. die Einstellung korrigiert.

Durch die Bestimmung der Temperatur des fluiden Produkts oder in der Nähe des fluiden Produkts und die Berücksichtigung dieser Temperatur bei der Dosiseinstellung kann die Abgabegenauigkeit eines Wirkstoffs an einen Patienten deutlich verbessert werden. Eine Unter- oder Überdosierung kann verhindert werden. Dadurch ist es möglich, den Erfolg einer Therapie oder das Wohlbefinden eines Patienten deutlich zu erhöhen.

Eine bevorzugte Ausführungsform der vorliegenden Erfindung wird von einer Verabreichungsvorrichtung in Form einer tragbaren Insulinpumpe gebildet, bei der wenigstens ein Temperatursensor innerhalb einer Kammer in der Insulinpumpe angeordnet ist, in die eine Ampulle eingesetzt werden kann, welche mit dem fluiden Produkt befüllt ist. Der Temperatursensor grenzt vorteilhafterweise unmittelbar an die Ampulle an. Es wäre auch möglich, die Kammer derart auszubilden, dass das fluide Produkt direkt in die Kammer eingefüllt werden kann, in diesem Fall kommt der Temperatursensor unmittelbar mit dem fluiden Produkt in Berührung. Ein weiterer Temperatursensor kann außen an der Verabreichungsvorrichtung, z. B. an einem Vorrichtungsgehäuse, angeordnet werden. Als Temperatursensoren können herkömmliche Sensoren verwendet werden, die als Massenprodukt kostengünstig erhältlich sind.

Vorzugsweise weist die erfindungsgemäße Verabreichungsvorrichtung eine Alarmeinrichtung auf, die bei Überschreiten einer vorbestimmten Temperatur, bzw. einer vorbestimmten Temperaturdifferenz bezüglich einer einstellbaren Referenztemperatur, ein Alarmsignal abgibt. Das Alarmsignal kann z. B. durch ein akustisches, visuelles und/oder vibratorisches Signal gegeben sein. Wird der Steuereinheit von dem Temperatursensor ein Temperatursignal übermittelt, das eine vorbestimmte Temperatur, bzw. eine vorbestimmte Temperaturdifferenz überschreitet, kann die Dosiereinrichtung zur Anpassung einer Produktdosis automatisch angesteuert werden, oder der Anwender kann aufgrund des Alarmsignals entscheiden, ob er eine Dosisänderung wünscht oder nicht.

In einer besonders bevorzugten Ausführungsform weist die Verabreichungsvorrichtung eine Überwachungseinrichtung zur Überwachung einer Okklusion oder einer Leckage innerhalb der Vorrichtung auf, die Sensoren innerhalb der Verabreichungsvorrichtung zur Überwachung umfasst. Solche Okklusions- bzw. Leckagesensoren basieren auf der Messung einer Kraft oder eines Drucks, der auf das fluide Produkt oder die Abgabeeinrichtung wirkt, bzw. messen die von der Abgabeeinrichtung zur Verabreichung notwendige Kraft. Die Messwerte der Sensoren werden an die Steuereinheit übermittelt, die bei Überschreiten eines Grenzwertes der Messwerte eine Verabreichung des fluiden Produkts mit der Verabreichungsvorrichtung unterbricht oder unterbindet, bzw. ein Alarmsignal abgibt. Die Messwerte derartiger Sensoren sind jedoch von der Temperatur des fluiden Produkts abhängig, d. h. die zur Verabreichung aufzuwendende Kraft oder der in dem Behälter herrschende Druck schwankt mit der Temperatur. Erfindungsgemäß können die von der Überwachungseinrichtung verwendeten Grenzwerte zur Überwachung einer Okklusion oder Leckage in Abhängigkeit der Temperatur des fluiden Produkts und/oder in Abhängigkeit von der Temperatur der Umgebung nahe des Produktbehälters eingestellt oder korrigiert werden.

Bei dem erfindungsgemäßen Verabreichungssteuerverfahren wird die Dosismenge einer Produktdosis in Abhängigkeit von einer Temperaturdifferenz bezüglich einer Referenztemperatur eingestellt. Als Referenztemperatur kann z. B. Raumtemperatur oder die Temperatur, die am Körper eines Patienten an der Stelle herrscht, an der er ein Verabreichungsgerät trägt, verwendet werden. Bei einer bestimmten Abweichung von diesem Referenzwert, d. h. beim Überschreiten einer bestimmten Temperaturdifferenz, kann dann eine für die Referenztemperatur korrekte Produktdosiseinstellung entsprechend der Höhe der Temperaturdifferenz angepasst, bzw. korrigiert werden, um die für eine erfolgreiche Behandlung des Patienten erforderliche Produktmenge bei einer Verabreichung abzugeben. Die vorbestimmte Dosismenge wird demnach derart geändert, dass die gewünschte Menge auch tatsächlich abgegeben wird. Beim Überschreiten einer Temperaturdifferenz kann von der Alarmeinrichtung ein Alarmsignal abgegeben werden, so dass der Anwender selbstständig eine Korrektur auslösen oder durchführen kann. Erfindungsgemäß wird die Dosiskorrektur automatisch von dar Steuereinheit veranlasst. Die Dosiseinstellung und die Korrektur können dabei auf einer Anzeige angegeben werden. Die Anzeige der Korrektur ist jedoch nicht unbedingt erforderlich, da durch die Korrektur die auf der Anzeige angegebene gewünschte Dosismenge eingehalten wird.

Erfindungsgemäß erfolgt eine Korrektur der Dosismenge bei Überschreiten der Temperaturdifferenz innerhalb eines vorbestimmten Zeitraums. Bei nur kurzzeitigen Schwankungen der Temperatur und damit Überschreitungen der vorgegebenen Temperaturdifferenz, bzw. der Referenztemperatur, kann es sein, dass keine Korrektur notwendig ist. Zum Beispiel erfolgt eine Zustandsänderung des fluiden Produkts innerhalb des Behälters erst dann, wenn eine erhöhte oder erniedrigte Temperatur über eine gewisse Zeit auf das Produkt einwirkt. Es ist daher sinnvoll, bei der Steuerung der Produktverabreichung die zeitliche Veränderung der Temperatur zu berücksichtigen.

Die Erfindungsidee wurde anhand einer Verabreichungsvorrichtung mit einem offenen System, d. h. es wird ein Fluid aus der Vorrichtung abgegeben, veranschaulicht. Grundsätzlich ist es jedoch auch denkbar, die Merkmale der vorliegenden Erfindung bei einem geschlossenen System einzusetzen, beispielsweise bei einem Messverfahren mit einem geschlossenen Fluidkreislauf, bei dem als Fluid eine Messflüssigkeit verwendet wird.

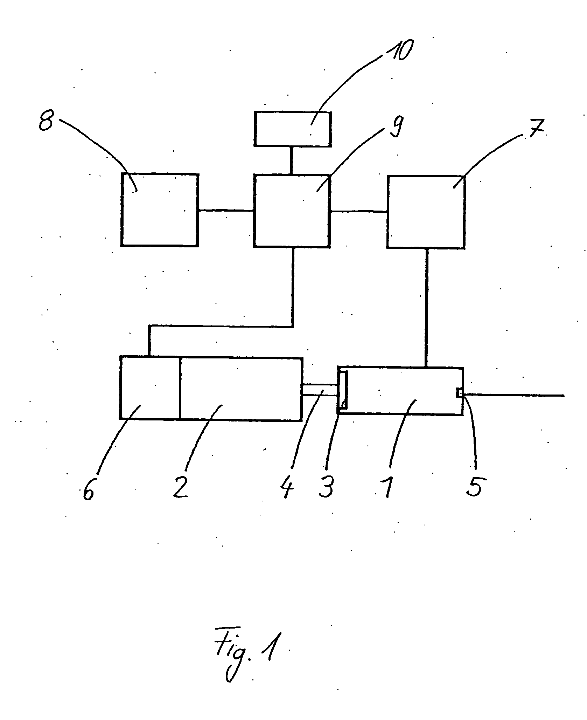

Die Erfindung wird anhand eines Ausführungsbeispiels durch die Zeichnung mit einer schematischen Darstellung einer Verabreichungsvorrichtung erläutert.

Eine Vorrichtung zur Verabreichung eines fluiden Produkts, wie sie in der Zeichnung gezeigt ist, weist einen Behälter 1 für das fluide Produkt, und eine Abgabeeinrichtung 2 zur Verabreichung des fluiden Produkts aus dem Behälter 1 auf. Der Behälter 1 ist derart ausgebildet, dass er eine Ampulle mit dem fluiden Produkt aufnehmen kann. Hierfür ist in dem Behälter 1 ein Stopfen 3 angeordnet, der von einem Abtriebsglied 4 der Abgabeeinrichtung 2 in Richtung auf einen Auslass 5 des Behälters 1 vorgeschoben wird. Weiter umfasst die Verabreichungsvorrichtung eine Dosiereinrichtung 6 zur Einstellung einer Dosismenge einer Produktdosis des fluiden Produkts. An einer Innenwand des Behälters 1 ist ein Temperatursensor 7 angeordnet, der bei eingeführter Ampulle neben dieser zu liegen kommt. Weiter weist die Verabreichungsvorrichtung eine Überwachungseinrichtung 8 mit einem Okklusions- und Leckagesensor zur Überwachung einer Okklusion und/oder Leckage innerhalb der Verabreichungsvorrichtung auf.

Der Temperatursensor 7, der Okklusions- und Leckagesensor, die Dosiereinrichtung 6 und die Abgabeeinrichtung 2 sind an eine Steuereinheit 9 angeschlossen. Die Steuereinheit 9 umfasst wenigstens einen Speicher auf, z. B. zur Speicherung bestimmter Verabreichungs- oder Patientenparameter. Der von dem Temperatursensor 7 in dem Behälter 1, d. h. nahe des fluiden Produkts, gemessene Temperaturwert wird an die Steuereinheit 9 abgegeben. Die Steuereinheit 9 korrigiert in Abhängigkeit der vom Sensor 7 gemessenen Temperatur eine Dosiseinstellung an der Dosiseinrichtung 6, bzw. die Verabreichung des fluiden Produkts durch die Abgabeeinrichtung 2. D. h. der vom Stopfen 3 für die Verabreichung zurückgelegte Vorschubweg wird entsprechend der gemessenen Temperatur verändert. Weiter werden von der Steuereinheit entsprechend der vom Temperatursensor 7 gemessenen Temperatur die zur Überwachung einer Okklusion oder Leckage verwendeten Grenzwerte der Überwachungseinrichtung 8 angepasst.

Eine Anzeige 10 dient zum Anzeigen verschiedener Parameter der Verabreichungsvorrichtung, wie einer Dosiseinstellung, und kann ein visuelles Warnsignal beim Überschreiten einer vom Temperatursensor 7 gemessenen Temperaturdifferenz angeben. Beim Anzeigen einer Warnung kann ein Anwender über Bedienknöpfe eine Dosiskorrektur veranlassen.

## Patentansprüche

1. Vorrichtung zur Verabreichung eines fluiden Produkts umfassend:
a) einen Behälter (1) für das fluide Produkt,
b) eine Abgabeeinrichtung (2) zur Verabreichung des fluiden Produkts aus dem Behälter (1) und
c) eine Dosiereinrichtung (6) zur Einstellung einer Produktdosis bei einer frei wählbaren Referenztemperatur,
d) wenigstens einen Temperatursensor (7) zur Messung der Temperatur des fluiden Produkts und/oder in der Nähe des fluiden Produkts,
e) eine Steuereinheit (9), die wenigstens mit dem Temperatursensor (7) und der Dosiereinrichtung (6) verbunden ist, wobei
f) die Steuereinheit (9) wenigstens einen Speicher zur Abspeicherung von Referenztemperaturdaten umfasst, und
g) die Steuereinheit (9) eine Dosiereinstellung an der Dosiereinrichtung (6) in Abhängigkeit der vom Temperatursensor (7) gemessenen Temperaturdaten automatisch korrigiert,
**dadurch gekennzeichnet, dass** die Steuereinheit (9) bei der Steuerung der Produktverabreichung die zeitliche Veränderung der Temperatur berücksichtigt und eine Korrektur der Dosiereinstellung erfolgt, wenn eine erhöhte oder erniedrigte Temperatur über eine gewisse Zeit auf das Produkt einwirkt.

2. Verabreichungsvorrichtung nach Anspruch 1, wobei der wenigstens eine Temperatursensor (7) an oder in dem Behälter (1) angeordnet ist.

3. Verabreichungsvorrichtung nach Anspruch 1 oder 2, wobei eine Alarmeinrichtung vorgesehen ist, die bei Überschreiten einer Temperaturdifferenz bezüglich einer Referenztemperatur ein Alarmsignal abgibt.

4. Verabreichungsvorrichtung nach dem vorhergehenden Anspruch, wobei das Alarmsignal ein akustisches, visuelles und/oder vibratorisches Alarmsignal ist.

5. Verabreichungsvorrichtung nach einem der vorhergehenden Ansprüche, wobei eine Überwachungseinrichtung (8) zur Überwachung einer Okklusion und/oder Leckage der Verabreichungsvorrichtung in Abhängigkeit der vom Temperatursensor (7) gemessenen Temperatur einstellbar ist.

## Claims

1. A device for administering a fluid product, comprising:
a) a container (1) for the fluid product;
b) a dispensing means (2) for administering the fluid product from the container (1); and
c) a dosing means (6) for setting a product dosage at a freely selectable reference temperature;
d) at least one temperature sensor (7) for measuring the temperature of the fluid product and/or the temperature in the vicinity of the fluid product;
e) a control unit (9) which is connected at least to the temperature sensor (7) and the dosing means (6), wherein
f) the control unit (9) comprises at least one memory for storing reference temperature data, and
g) the control unit (9) automatically corrects a dosage setting on the dosing means (6) in accordance with the temperature data measured by the temperature sensor (7),
**characterised in that** the control unit (9) takes into account the change in temperature over time when controlling the administering of product, and the dosage setting is corrected when an increased or reduced temperature acts on the product over a certain period of time.

2. The administering device according to claim 1, wherein the at least one temperature sensor (7) is arranged on or in the container (1).

3. The administering device according to claim 1 or 2, wherein an alarm means is provided which outputs an alarm signal when a temperature difference with respect to a reference temperature is exceeded.

4. The administering device according to the preceding claim, wherein the alarm signal is an acoustic, visual and/or vibration alarm signal.

5. The administering device according to any one of the preceding claims, wherein a monitoring means (8) for monitoring an occlusion and/or leak in the administering device can be set in accordance with the temperature measured by the temperature sensor (7).

## Revendications

1. Dispositif pour administrer un produit fluide, comportant :
a) un conteneur (1) pour le produit fluide,
b) un dispositif de distribution (2) pour administrer le produit fluide à partir du conteneur (1) et
c) un dispositif de dosage (6) pour ajuster une dose de produit à une température de référence librement sélectionnable,
d) au moins un capteur de température (7) pour mesurer la température du produit fluide et/ou à proximité du produit fluide,
e) une unité de commande (9) au moins reliée au capteur de température (7) et au dispositif de dosage (6), dans lequel
f) l'unité de commande (9) comporte au moins une mémoire pour mémoriser des données de température de référence, et
g) l'unité de commande (9) corrige automatiquement un réglage de dosage sur le dispositif de dosage (6) en fonction des données de température mesurées par le capteur de température (7),
**caractérisé en ce que** l'unité de commande (9) tient compte de la variation de la température dans le temps lors de la commande de l'administration du produit et effectue une correction du réglage de dosage lorsqu'une augmentation ou une diminution de température influe sur le produit pendant une certaine période de temps.

2. Dispositif d'administration selon la revendication 1, dans lequel le au moins un capteur de température (7) est agencé sur ou dans le conteneur (1).

3. Dispositif d'administration selon la revendication 1 ou 2, dans lequel est prévu un dispositif d'alarme qui génère un signal d'alarme lorsqu'une différence de température par rapport à une température de référence est dépassée.

4. Dispositif d'administration selon la revendication précédente, dans lequel le signal d'alarme est un signal d'alarme acoustique, visuel et/ou vibratoire.

5. Dispositif d'administration selon l'une quelconque des revendications précédentes, dans lequel un dispositif de surveillance (8) peut être réglé afin de surveiller une occlusion et/ou une fuite du dispositif d'administration en fonction de la température mesurée par le capteur de température (7).
